# EUROPÄISCHE PATENTSCHRIFT

(11) **EP 0 532 839 B1**
(45) Veröffentlichungstag und Bekanntmachung des Hinweises auf die Patenterteilung: **13.03.1996**
(21) Anmeldenummer: 92110014.5
(22) Anmeldetag: 13.06.1992
(51) Int. Cl.: A23B 9/02, A23L 3/3418, A23L 3/16, A23L 3/18, A23L 3/015, A23B 4/005, A23B 7/005

(54) **Verfahren und Vorrichtung zum Entkeimen eines Produktes**
Process and apparatus for sterilising
Procédé et dispositif pour la stérilisation d'un produit

(30) Priorität: 27.06.1991 CH 1326/91
(43) Veröffentlichungstag der Anmeldung: 24.03.1993
(73) Patentinhaber: MAVAG VERFAHRENSTECHNIK AG, CH-8852 Altendorf (CH)
(72) Erfinder: MAVAG VERFAHRENSTECHNIK AG, CH-8852 Altendorf (CH)

(56) Entgegenhaltungen:
- EP-A- 0 006 369
- EP-A- 0 196 464
- EP-A- 0 269 257
- CH-A- 406 810
- US-A- 3 721 527
- US-A- 3 939 287
- US-A- 4 924 071

## Beschreibung

Die Erfindung betrifft ein Verfahren und eine Vorrichtung zum Entkeimen eines schüttbaren Produktes mit den Verfahrensschritten gemäss Oberbegriff des ersten Verfahrensanspruches und Oberbegriff des ersten Vorrichtungsanspruches.

Aus dem Stand der Technik sind Verfahren und Vorrichtungen zum Entkeimen von schüttbaren Produkten bekannt, beispielsweise zeigt die Europäische Patentanmeldung mit der Veröffentlichungsnummer 0196464 A3 ein Verfahren, um pulverförmige und/oder granulöse Substanzen zu erwärmen. Die Erwärmung erfolgt mittels eines Heissluftstromes, welcher durch ein Heizrohr strömt, welches einerseits einen Heizmantel für das Erwärmen der Rohrwände sowie anderseits im Rohr selbst Leitschaufeln umfasst, welche dafür sorgen, dass die Strömung samt der pulverigen und/oder granulösen Substanz im Rohr verwirbelt wird. Das Ausscheiden des erwärmten Produktes aus dem Heissluftstrom geschieht mittels eines Zyklons.

Ein weiterer Stand der Technik ist aus der Europäischen Patentanmeldung mit der Veröffentlichungsnummer 026925 A2 bekannt, in welchem granulöse und blätterige Gewürze sterilisiert werden. Dazu werden diese granulösen oder blätterigen Gewürze einem Rührwerkmischer zugeführt, welcher einerseits einen geheizten Mantel aufweist und in welchen andererseits während des Mischens Dampf eingeblasen wird.

Nach einer vorgegebenen Zeit wird das sterilisierte Produkt einem weiteren Rührwerkmischer zugeführt, in welchem das Produkt während des Mischens gekühlt wird. Als Kühlmittel dienen die gekühlten Wände des Mischers.

Der Transport des Produktes aus einem Vorratbehälter in den Sterilisationsmischer sowie vom Sterilisationsmischer in den Kühlmischer geschieht je indem der jeweilige Mischer in Unterdruck versetzt wird, bevor der Produkteeingang zum jeweiligen Mischer geöffnet wird. Hingegen geschieht der Transport aus dem Kühlmischer durch einen im Kühlmischer aufgebauten Überdruck.

In einem weiteren aus der US-Patentschrift Nr.3721527 bekannten Stand der Technik wird ein zu sterilisierendes Schüttgut mittels eines Dosierelements in einen Rührwerkmischer gefördert, anschliessend mit einer vorgegebenen Menge eines Sterilisiermediums erwärmt und im weiteren mit gesättigtem Dampf beaufschlagt. Während der ganzen Zeit des Sterilisierens ist das Rührwerk in Bewegung. Die Sterilisierdauer beträgt beispielsweise 3 Minuten.

Im Anschluss daran wird das Rührwerk gestoppt und das Sterilisiermedium evakuiert. Anschliessend wird der Behälter mittels einer Vakuumleitung in Unterdruck versetzt, um das Material zu trocknen. Gleichzeitig wird das Rührwerk wieder in Gang gesetzt. Zusätzlich wird der Mantel des Mischers mittels Dampferwärmt. Letztlich wird das Produkt mittels steriler Luft ventiliert und in einen zweiten sterilen Mischer gefördert.

In einer Variante kann das Trocknen auch im vorgenannten zweiten Mischer erfolgen.

Der Nachteil dieses genannten Standes der Technik besteht darin, dass Rührwerkmischer oder irgendwelche Hilfsmittel notwendig sind, um die relativ grossen Mengen an zu sterilisierendem Produkt während des Sterilisationsprozesses zu durchmischen , damit eine gleichmässige Sterilisation erfolgt. Dabei liegt der Nachteil nicht im eigentlichen Mischeffekt, sondern darin, dass leicht beschädigbare Produkte durch die mechanische Beanspruchung des Rührwerkmischers oder der Leitschaufeln beschädigt werden können, was insbesondere dann schädlich ist, wenn es sich um Produkte handelt, die in gebrochenem Zustand leicht oxydieren oder welche in blätteriger oder stengliger Form schonend sterilisiert werden sollten, um möglichst wenig oder keinen Ausschuss während des Sterilisierens zu produzieren. Ausserdem gelangt der Dampf nicht an die gesamte Produkteoberfläche, was sich in relativ hohen Restkeimzahlen ausdrückt.

Stand der Technik ist auch die Anwendung der Kondensationsenergie von Wasserdampf zum Kochen von frischem Gemüse, Früchten, Fleisch etc. Die europäische Patentanmeldung mit der Veröffentlichungsnunmer 0 006 369 A1 beschreibt ein verfeinertes Verfahren zum partiellen bis vollständigen Kochen indem der Behälter vor und nach dem Kochen unter Vakuum gesetzt wird. Verarbeitet wird wie beschrieben im genannten Behälter z.B.
- eine Füllung von 1000 kg Kartoffelstücken von 10 bis 20 mm Grösse zum partiellen Vorkochen während 20 Minuten notwendiger Kochzeit
- eine Füllung mit grünen Erbsen zum Blanchieren.

Durch das Vorvakuum und Verwendung der Kondensationsenergie wird eine kürzere Kochzeit erreicht als bei früheren Verfahren, durch das Nachvakuum Rückkühlung im gleichen Behälter, was Kontaminationsprobleme durch einen separaten Kühler ausschliesst. Der eintretende Dampf trifft auf frisches, ungetrocknetes, ungedarrtes Produkt mit hohem Wassergehalt und kein Wasserdampfkondensat aufnehmend, sowie teilweise freiem Wasserfilm auf der Produktoberfläche, Kondensat das sich zwischen dem Produkt und der Behälterwand bildet und gelangt sich abkühlend ins Innere des Produkthaufens. In diesem Zustand ist das Produkt der Behälterfläche entlang teilweise oberflächlich vorgekocht resp.blanchiert, weiter gegen das Innere des Produkthaufens jedoch nicht behandelt. Durch Wärmezufuhr mittels der beheizten Behälterinnenfläche wie beispielsweise beschrieben während 20 Minuten wird nun ein Kochprozess eingeleitet, während welchem im ganzen Produkthaufen eine annähernd gleichmässige Temperatur erreicht wird. Bekannterweise wird während dieser Kochzeit das Produkt erwärmt, Wirkstoffe wie z.B. ätherische Öle verflüchtigt oder zerstört und der Geschmack, Geruch, Farbe etc verändert. Wird anderseits dieser Kochprozess nichtvollständig durchgeführt, ist das Produkt im Produkthaufen ungenügend bis nicht vorgekocht resp. sterilisiert.

Die Aufgabe der Erfindung bestand deshalb darin, das Sterilisieren derart schonend durchzuführen, dass im wesentlichen kein Ausschuss erzeugt wird und andererseits trotzdem die einzelnen Teile des Produktes gleichmässig sterilisiert werden. Die Aufgabe wird durch die im Kennzeichen des ersten Verfahrens- und des ersten Vorrichtungsanspruches aufgeführten Merkmale gelöst. Die abhängigen Ansprüche zeigen weitere vorteilhafte Ausführungsformen.

Die Erfindung ist mit Hilfe der folgenden Figuren beispielsweise beschrieben.

Es zeigen:
- Fig. 1: eine erfindungsgemässe Sterilisationsanlage, halbschematisch dargestellt,
- Fig. 2 & 3: je eine Variante der Sterilisationsanlage von Fig. 1
- Fig. 4: ein Detail der Sterilisationsanlage von Fig.2, vergrössert dargestellt,
- Fig. 5: eine Draufsicht des Details von Fig.4, im Schnitt gemäss den Schnittlinien I dargestellt.

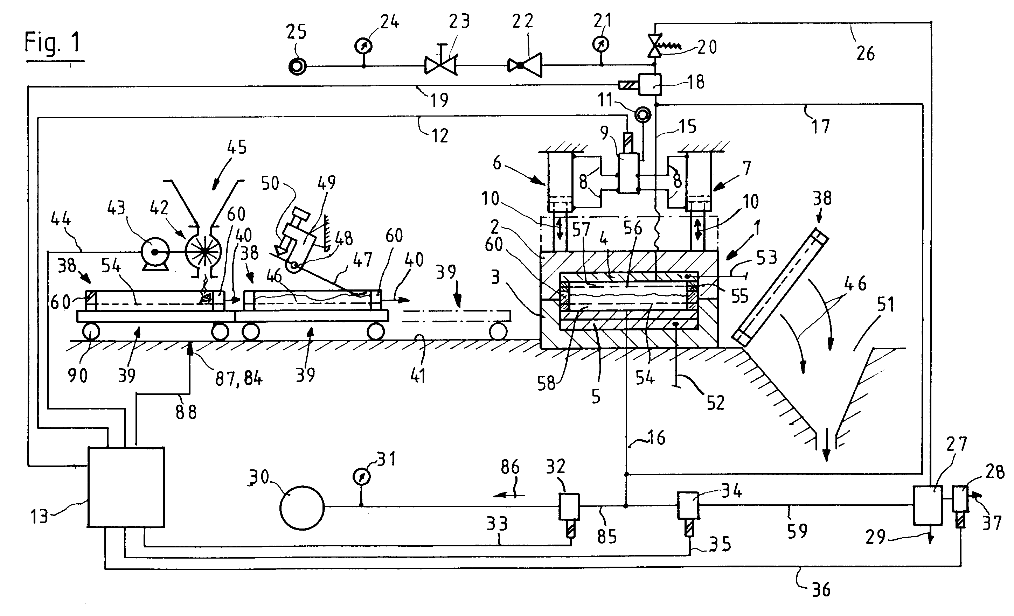

Die Figur 1 zeigt einen autoklav-artigen Behälter 1 mit einem muldenförmigen, abhebbaren Oberteil 2 und einem muldenförmigen stationären Unterteil 3.

Im Oberteil 2 ist ein Heizelement 4 und im Unterteil 3 ein Heizelement 5 vorgesehen. Diese Heizelemente können entweder elektrisch oder dampfbezeizte Elemente sein.

Im weiteren ist im Oberteil 2 ein Deckelrahmen 55 an das Heizelement 4 angrenzend eingelassen, welcher mit einer Gewebedecke 56 versehen ist.

Ein zu diesem Deckelrahmen 55 deckungsgleich vorgesehener Gefässrahmen 60 ist im Unterteil 3 eingelassen.

Der Gefässrahmen 60 ist mit einem Gewebeboden 54 versehen. Diese Gewebe können entweder Woll- oder Baumwollgewebe sein. Die Energiezufuhrleitung für das Heizelement 4 ist mit 54 und die Energiezufuhrleitung für das Heizelement 5 ist mit 53 gekennzeichnet.

Das Öffnen und wieder Schliessen in den Bewegungsrichtungen 10 des Oberteiles 2 geschieht mit den Hubzylindern 6 und 7, welche über Steuerleitungen 8 von einem Steuerventil 9 mittels eines Druckmediums gesteuert werden. Das Steuerventil 9 ist seinerseits ein Elektroventil und wird mittels der Steuerleitung 12 von einer Steuerung 13 gesteuert. Die Druckmediumleitung 11 liefert das notwendige Druckmedium an das Steuerventil 9. Die Hubzylinder 6 und 7 sind stationär angeordnet. Der abgehobene Oberteil 2 ist mit strichpunktierten Linien angedeutet.

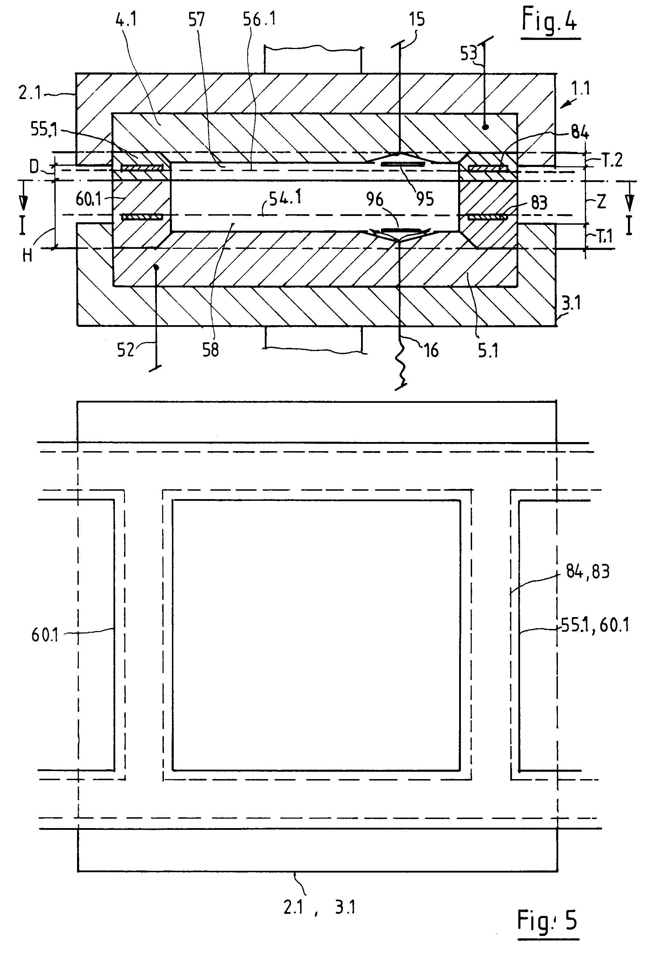

Betreffend die Details des Deckelrahmens 55 und des Gefässrahmens 60 wird auf die Beschreibung im Zusammenhang mit den Figuren 4 und 5 verwiesen.

Mittels einer oberen Dampfleitung 15 wird Dampf in den sich oberhalb der Gewebedecke 56 befindlichen Raum 57 und mittels einer unteren Dampfleitung 16 wird Dampf in den sich unterhalb des Gewebebodens 54 befindlichen Raum 58 eingeblasen. Dieser Dampf weist einen Druck zwischen 1,5 und 7 bar auf und weist eine Temperatur von 110° bis 165° Celsius auf.

Der in die Räume 57 und 58 eingeblasene Dampf verteilt sich in diesen Räumen und tritt einerseits durch die Gewebedecke 56 und anderseits durch den Gewebeboden 54 durch in die sich auf dem Gewebeboden 54 befindliche zu sterilisierende Produktschicht. Die eigentlichen Verfahrensschritte werden später beschrieben.

Das Absperren des Dampfes in der Leitung 15 geschieht durch ein Dampfsteuerventil 18, welches von der Steuerung 13 über die Steuerleitung 19 elektrisch gesteuert wird. In der Dampfleitung 15 ist zwischen einer Dampfzufuhrleitung und dem vorgenannten Dampfsteuerventil 18, in Dampfströmrichtung gesehen, zuerst ein Manometer 24, dann ein Absperrventil 23, anschliessend ein Druckreduzierventil 22 und schliesslich nochmals ein Manometer 21 vorgesehen.

Vor dem Dampfsteuerventil 18, in Dampfströmungsrichtung gesehen, zweigt eine Dampfabströmleitung 26 ab, welche in einen Dampfkondensator 27 mündet. In dieser Leitung 26 ist ein Überdruckventil 20 eingebaut.

Am Dampfkondensator 27 ist für den Wasserablass ein Ventil 28 mit einer Entsorgungsleitung 37 vorgesehen, welches von der Steuerung 13 mittels einer Steuerleitung 36 elektrisch gesteuert wird. Mit 29 ist der Umgebungsluftzutritt des Dampfkondensators 27 gekennzeichnet.

Um den Dampf und das anschliessend erwähnte Vakuum Von der oberen Dampfleitung 15 in die untere Dampfleitung 16 zu transferieren, ist eine Verbindungsleitung 17 vorgesehen.

Im weiteren kann in der Dampfleitung 15 und 16 je ein Vakuum erzeugt werden, um die vorangehend mit Dampf beaufschlagten Räume in Unterdruck zu versetzen.

Um das Letztgenannte zu ermöglichen, ist die Dampfleitung 16 mit einer Unterdruckleitung 85 verbunden, welche ihrerseits mit einer Vakuumpumpe 30 verbunden ist.

In der Unterdruckleitung 85 ist in der Strö mungsrichtung 86 des in Unterdruck stehenden Mediums ein Steuerventil 32 sowie ein Manometer 31 vorgesehen. Das Steuerventil 32 ist mittels einer Steuerleitung 33 mit der Steuerung 13 verbunden. Im weiteren ist die untere Dampfleitung 16 noch mittels einer Dampfabströmleitung 59 mit dem Dampfkondensator 27 verbunden und weist zwischen dem Anschluss an die Dampfleitung 16 und dem Kondensator 27 ein Steuerventil 34 auf, welches mittels Steuerleitung 35 mit der Steuerung 13 verbunden ist.

Das Verfahren zur Sterilisation des früher genannten Produktes geschieht folgendermassen: Ein Rahmengefäss 38 wird auf einen Verschiebewagen 39 gelegt, welcher in der Verschieberichtung 40 auf einer Verschiebebahn 41 bewegt werden kann.

In der erstgezeigten Position, von links nach rechts, in Verschieberichtung 40 gesehen, liegt der Verschiebewagen 39 derart, dass ein aus einem Vorratsbehälter 45 und durch ein Dosierelement 42 auf, den Gewebeboden 54 des Rahmengefässes 38 eingespeistes Produkt durch Verschieben des Verschiebewagens 39 in der Richtung 40 im wesentlichen gleichmässig auf den Gewebeboden 54 verteilt wird.

Im gezeigten Beispiel handelt es sich um eine Dosierschleuse; es versteht sich jedoch, dass andere Dosierelemente, beispielsweise Vibro-Dosierelemente, Dosierbandwaagen ebenfalls Verwendung finden können.

Die Dosierschleuse 42 wird von einem Antriebsmotor 43 angetrieben, welcher über die Steuerleitung 44 mit der Steuerung 13 verbunden ist.

Das Dosieren mittels einem Dosierelement 42 wird unterbrochen, wenn ein in der Verschiebebahn 41 eingelassener Sensor 87 feststellt, dass ein daraufstehendes Rad 90 des Wagens 39 den Sensor berührt. Der Sensor meldet dies via Steuerleitung 88 der Steuerung 13, die den Motor 43 über die Steuerleitung 44 stoppt.

Um eine vorgegebene Menge an Produkt gleichmässig im Rahmengefäss 38 eingespeist zu erhalten, muss der Verschiebewagen 39 den entsprechenden Weg innerhalb einer vorgegebenen, der Dosiergeschwindigkeit entsprechenden Zeit durchlaufen haben. Dies kann, falls die Verteilung des eingespeisten Produktes manuell geschieht, auf Grund der Erfahrung der Bedienungsperson geschehen oder es kann eine Vorschubvorrichtung vorgesehen werden (nicht gezeigt), welche den Wagen mit einer vorgegebenen Geschwindigkeit bis zum Ansprechen des Sensors 84 verschiebt.

Das anschliessende Weiterverschieben des Verschiebewagens 39 in die zweite in Fig.1 gezeigte Position, in welcher die Oberfläche des eingefüllten Produktes mit einem Verteilelement 47 geglättet wird und weiter in die mit strichpunktierten Linien gezeigte Position kann entweder manuell oder mit einer weiteren Verschiebevorrichtung (nicht gezeigt) verschoben werden.

Das Verteilelement 47 ist mittels einer Schwenkachse 48 schwenkbar an einem stationären Träger 49 gelagert, wobei die Lage des Verteilelementes 47 durch einen verstellbaren Anschlag 50 gegeben ist. Das Verteilelement 47 ist rechts von der Schwenkachse 48, mit Blick auf Fig.1 gesehen, schwerer als links von der Schwenkachse, dh. dass das Verteilelement 47 mittels des entsprechenden Eigengewichtes auf die Oberfläche des eingeschütteten Produktes drückt und dieses infolge des Verschiebens des Verschiebewagens 39 glättet.

Als nächster Schritt wird das Rahmengefäss 38 in den mittlerweile geöffneten autoklav-artigen Behälter 1 eingelegt.

Das Schliessen geschieht, wie bereits früher beschrieben, wiederum mittels eines die Verfahrensschritte durchführenden Steuerprogramms der Steuerung 13, wobei dies durch einen Druckknopf (nicht gezeigt) an der Steuerung gestartet wird.

Beim Verschliessen des Behälters 1 wird der Deckelrahmen 55 auf Gefässrahmen 60 gepresst und mechanisch verriegelt, sodass dadurch der Innenraum dicht verschlossen ist.

Zum ersten sind in diesem Zustand die Steuerventile 18, 32 und 34 geschlossen.

Als erster Verfahrensschritt wird nun das Steuerventil 32 geöffnet, um über die Leitungen 85, 16, 17 und 15 einen Unterdruck im vorgenannten Innenraum zu erzeugen.

Nach einer vorgegebenen Zeit wird das Steuerventil 32 geschlossen und das Steuerventil 18 geöffnet, sodass Dampf mit vorgegebenem Druck in den evakuierten Innenraum einströmen kann. Auf diese Weise entsteht der Vorteil, dass sich der Dampf innert kürzester Zeit und gleichmässig, ohne Luftverdrängung, im Innenraum sowie auch in der zu sterilisierenden Schicht verteilt.

Die Wahl des Dampfdruckes innerhalb der vorgenannten 1,5 - 7bar sowie die Temperatur zwischen den vorgenannten 110°-165° Celsius geschieht aufgrund empirischer Versuche je nach dem zu sterilisierenden Produkt und der zu sterilisierenden Menge.

Nach dem Schliessen des Ventiles 18 nach der vorgenannten vorgegebenen Zeit wird das Steuerventil 34geöffnet, sodass der Dampf über die Dampfabströmleitung 59 in den Kondensator 27 gelangt. Die Steuerung 13 betätigt in gewählten Zeitabständen für kurze Zeit über die Steuerleitung 36 das Ventil 28, und das Kondensat fliesst über die Leitung 37 ab.

Im Anschluss daran wird das Steuerventil 34 wieder geschlossen und das Steuerventil 32 wieder geöffnet, sodass mittels des neuerdings im Innenraum erzeugten Unterdruckes das Produkt gekühlt werden kann. Dies geschieht ebenfalls während einer vorgegebenen, empirisch ermittelten Dauer.

Nach erneutem Unterbrechen des Unterdruckes durch das Steuerventil 32 wird das Steuerventil 34 geöffnet, sodass der Unterdruck im vorgenannten Innenraum abgebaut und der Behälter 1 wieder geöffnet werden kann, um das Rahmengefäss 38 herauszunehmen, sodass das Produkt 46 durch Kippen des Rahmengefässes 38 in eine Entleertrimelle 51 geschüttet werden kann.

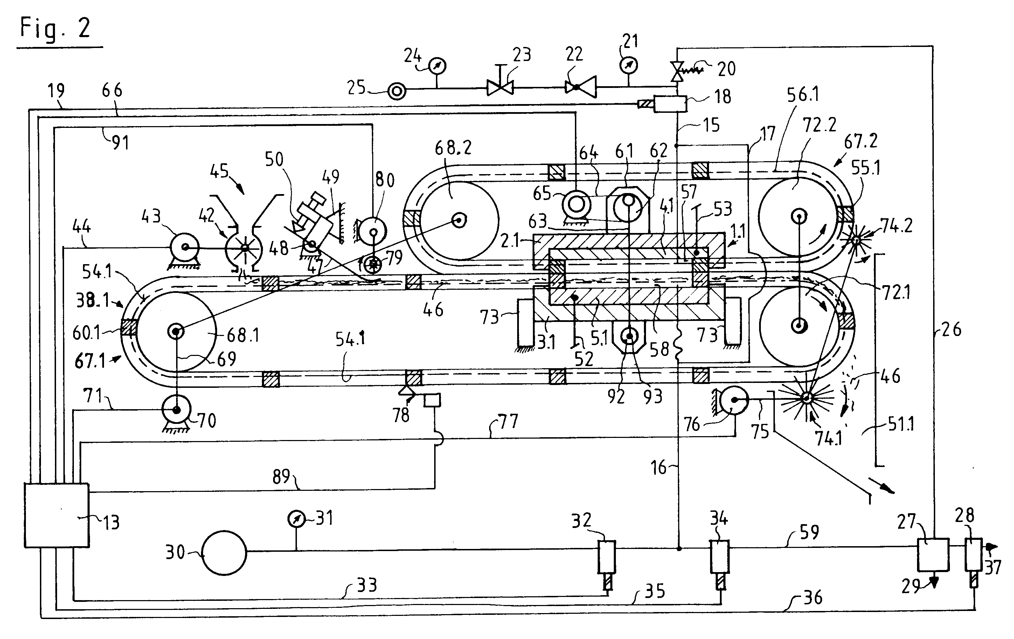

Die Figur 2 zeigt insofern eine Variante zu Fig.1, als in Fig.2 die Rahmengefässe 38.1 und die Deckelrahmen zu endlosen Transportbändern 67.1/67.2 zusammengebracht sind, welche einerseits von angetriebenen Umlenkrollen 68.1/68.2 und andererseits von frei drehenden Umlenkrollen 72.1/72.2 aufgenommen sind.

Die angetriebenen Umlenkrollen 68.1, 68.2 werden mittels eines Übertriebes 69 durch einen Antriebsmotor 70 angetrieben, welcher mittels einer Steuerleitung 71 von der Steuerung 13 gesteuert wird.

Grundsätzlich haben diejenigen Elemente, welche in Fig.1 bereits aufgeführt sind, dieselben Bezugszeichen oder, falls sie dieselbe Funktion aufweisen, jedoch etwas modifiziert sind, ist das Bezugszeichen von Fig.1 in Fig.2 mit ".1" erweitert, zB. wird das Gehäuse in Fig.2 mit 1.1 bezeichnet.

Neu gegenüber der Anlage von Fig.1 ist eine Reinigungswalze 79, welche die Oberfläche jedes Gefässrahmens 60.1 reinigt bevor dieser Rahmenteil in die Lage im autoklav-artigen Behälter 1.1 versetzt wird. Diese Reinigungswalze wird durch einen Antriebsmotor 80 angetrieben, welcher mittels einer Steuerleitung 91 von der Steuerung 13 gesteuert wird. Grundsätzlich ist diese Reinigungswalze 79 während des ganzen Betriebes in Funktion.

Ebenfalls anders gelöst als in Fig.1 ist die Wahl des stationären Teiles des Behälters 1.1, indem hier der Oberteil 2.1 stationär angeordnet ist und der Unterteil 3.1 in stationären Gleitführungen 73 für das Öffnen des Behälters 1.1 verschiebbar geführt ist.

Das Öffnen selbst geschieht mittels Zugstangen 63, welche mittels drehbarem Achslager 92 an der am Unterteil 3.1 feststehenden Achse 93 befestigt sind.

Das obere Ende der Zugstangen 63, mit Blick auf Fig.2 gesehen, sind exzentrisch auf einer Welle 62 gelagert, welche ihrerseits in einem zum Oberteil 2.1 gehörenden Wellenlager 61 drehbar gelagert ist.

Die Welle 62 ist mittels Übertrieb 64 von einem Antriebsmotor 65 angetrieben, welcher mittels einer Steuerleitung 66 von der Steuerung 13 gesteuert wird.

Durch die exzentrische Lagerung des oberen Endes der Zugstangen 63 in der Welle 62 wird der Unterteil 3.1 beim Drehen der Welle 62 für das Öffnen des Behälters 1.1 nach unten verschoben und für das Schliessen wieder nach oben.

Ebenfalls zusätzlich gegenüber der Anlage von Fig.1 sind in Fig.2 Reinigungswalzen 74.1, 74.2 vorgesehen, welche mittels eines Übertriebes 75 von einem Antriebsmotor 76 angetrieben sind, der mittels einer Steuerleitung 77 von der Steuerung 13 gesteuert wird.

Die Reinigungswalzen 74.1, 74.2 umfassen, wie gezeigt, lange Borsten, welche bis auf den Gewebeboden 54.1 resp. 56.1 reichen, um diesen zu reinigen. Die Drehrichtung der Reinigungsbürste ist mit dem Pfeil angezeigt.

Die Verfahrensschritte sind grundsätzlich wie für Fig.1 beschrieben, lediglich dass die Rahmengefässe automatisch schrittweise um eine Stufe weiter gefördert werden, wobei jeweils ein Positionsschalter 78 die Position über eine Steuerleitung 89 der Steuerung 13 übermittelt.

Das Entleeren des sterilisierten Produktes erfolgt in die Entleerungstrimelle 51.1.

Als Variante zum Deckeltransportband 67.2 kann auch, wie in Fig.1 Pos.53 gezeigt, ein permanenter Deckelrahmen eingesetzt sein; dies insbesondere beim Behandeln von körnigen und stückigen Produkten.

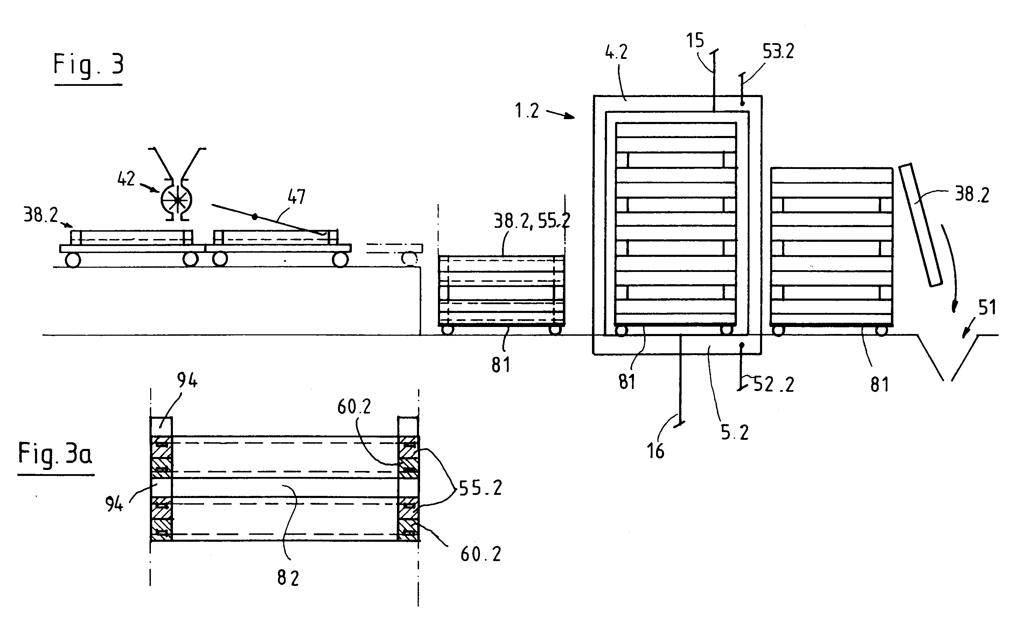

Fig.3 zeigt eine weitere Variante der Figur 1, indem jedoch die in Fig.1 gezeigten Rahmengefässe hier mit 38.2 gekennzeichnet sind, und zwar weil der Rahmen der Rahmengefässe am untern Rand (mit Blick auf Fig.3 gesehen) allseitig mit durchgehenden Öffnungen 82 versehen ist, um, wie später beschrieben, den Sterilisierdampf in den Innenraum der Rahmengefässe strömen zu lassen.

Aus Fig.3 ist ersichtlich, dass die Rahmengefässe 38.2 mit Deckelrahmen 55.2 auf einen Transportwagen 81 bis zu einer vorgegebenen Anzahl gestapelt werden, bevor diese mittels des Transportwagens 81 in einen geöffneten (nicht dargestellt) autoklav-artigen Behälter 1.2 eingeschoben werden.

Ein Unterschied zur Anordnung von Fig.1 besteht darin, dass die Rahmengefässe 38.2 durch Deckelrahmen 55.2 abgedeckt werden und die letzteren in den 4 Ecken mit Distanzstücken 94 versehen sind, sodass der durch die Leitungen 15 und 16 einströmende Dampf in die senkrechten Räume rund um den Stapel an Rahmengefässen 38.2/55.2 und durch die mit den Zapfen 94 gegebenen Gefässabstände gleichmässig in die Innenräume der Rahmengefässe 38.2 verteilt wird. Nach einer vorgegebenen Zeit wird der Autoklav geöffnet (nicht dargestellt), sodass der Transportwagen 81 nach rechts, mit Blick auf Fig.3 gesehen, aus dem Autoklaven 1.2 herausgefahren werden kann, um die einzelnen Rahmengefässe 38.2 in die Entleertrimelle 51 zu entleeren.

Der Einfachheit halber wurde in dieser Figur alles weggelassen, was steuerungsmässig gleich ist wie in Fig.1.

Fig.4 zeigt einen autoklav-artigen Behälter 1.1 von Fig.2, vergrössert dargestellt. Daraus ist ersichtlich, dass der von den Leitungen 15 und 16 ausströmende Dampf primär auf Prallplatten 95 und 96 aufprallt und dadurch besser in die Breite des Innenraumes verteilt wird.

Wie bereits früher erwähnt, sind die Skizzen halbschematisch, dh. dass selbstverständlich beispielsweise die Wahl der Lage der Dampfleitungen 15 und 16 besser, dh. optimaler gewählt werden kann, beispielsweise in eine zentralere Lage, was jedoch hier der Übersicht naher nicht getan wurde.

Im weiteren ist in dieser Figur wie auch in der Figur 5 gezeigt. dass der Deckelrahmen 55.1 mit Armiereinlagen 84 und der Gefässrahmen 60.1 mit Armiereinlagen 83 versehen sind. und zwar, um diesen Rahmenelementen in Längs- und Querrichtung eine grössere Stabilität und zusätzliche Festigkeit zu verleihen.

Grundsätzlich sind die Deckelrahmen 55, resp.55.1, wie auch die Gefässrahmen 60, resp.60.1 aus elastischem Material hergestellt, um mittels des Schliessdruckes, welcher beim Schliessen des autoklav-artigen Behälters 1 und 1.1 entsteht, einen nach aussen auch gegen den im Rahmen entstehenden Überdruck dicht abzuschliessen. Das heisst, dass die rahmenartige Stirnfläche des Oberteiles 2 und 2.1, resp. des Unterteiles 3 und 3.1 nicht aufeinander aufliegen müssen, um den Innenraum der Rahmengefässe abzudichten. In der Ausführung gemäss Fig.1 könnte allerdings auch eine Dichtung zwischen dem Oberteil 2 und dem Unterteil 3 vorgesehen werden, sodass in der Wahl des Materials für den Deckelrahmen 55 und den Gefässrahmen 60 mehr Freiheit besteht. Ein weiterer Unterschied zu Fig.1 besteht darin, dass das Gehäuse 1.1 den Deckelrahmen 55.1 und den Gefässrahmen 60.1 infolge des Transportbandkonzeptes nicht mehr ganz umschliesst. Aus Fig.4 ist ersichtlich, dass der eine Höhe H aufweisende Gefässrahmen mit einer Tiefe T.1 in den Unterteil 3.1 hineinragt und von diesem gegen den nach aussen gerichteten Dampfdruck abstützt. Dasselbe gilt für den Deckelrahmen 55.1, welcher mit einer Tiefe T.2 in den Oberteil 2.1 ragt. Damit bleibt ein Abstand D zwischen dem Gefässrahmen 60.1 und dem Oberteil 2.1 und eine Öffnung Z zwischen dem Oberteil 2.1 und dem Unterteil 3.1 in der Laufrichtung des Gefässtransportbandes offen, ohne dass der Dampfdruck in der Lage wäre, den Deckelrahmen 55.1 und den Gefässrahmen aus dem Unterteil 3.1 und dem Oberteil 2.1 zu drängen. Die Schliesskraft der Zugstangen 63 ist so bemessen, dass das Ausströmen von Dampf in die Atmosphäre verhindert wird.

Wie bereits früher erwähnt, ist die Gewebedecke 56.1 wie auch der Gewebeboden 54.1 aus einem Woll- oder Baumwollgewebe, das den wesentlichen Vorteil gegenüber beispielsweise einem Lochblech oder einem Stahldrahtgewebe aufweist, dass Wolle und Baumwolle die Fähigkeit haben, Flüssigkeit aufzunehmen resp. abzugeben und trotzdem dampfdurchlässig sind, dh. dass durch allfällige Abkühlung des Dampfes im Raum und daran anschliessender Kondensation das Kondensat von den Geweben aufgenommen werden kann und dadurch im Produkt Klumpenbildung aufgrund von Vermengung von Wassertropfen mit Produkt verhindert wird.

## Patentansprüche

1. Verfahren zum Entkeimen eines schüttbaren Produktes, mit den Verfahrensschritten:
- Einführen einer vorgegebenen Menge an zu entkeimenden Produktes in einen Behälter,
- Einblasen von Dampf von vorgegebener Temperatur und vorgegebenem Druck in den Behälter während einer vorgegebenen Zeitdauer,
- Entspannen des Dampfes durch Ablassen in die Atmosphäre,
- Behälter unter Vakuum setzen, um das Produkt zu kühlen,
- Behälter nach dem Einführen des Produktes und vor dem Einführen des Produktes und vor dem Einführen des Dampfes für das Evakuieren der sich darin befindlichen Luft in Unterdruck setzen.
dadurch gekennzeichnet,
dass das Produkt bereits als Schicht von vorgegebener Dicke in einem Gefäss in den Behälter eingeführt wird.

2. Verfahren nach Anspruch 1, dadurch gekennzeichnet, dass die Produktschicht auf einem wasseraufnahme- und wasserabgabefähigen dampfdurchlässigem Gewebe liegt.

3. Verfahren nach Anspruch 2, dadurch gekennzeichnet dass zusätzlich die Produktschicht von einem dampfdurchlässigem und wasseraufnahme- und wasserabgabefähigen Gewebe abgedeckt wird.

4. Verfahren nach Anspruch 1, dadurch gekennzeichnet, dass der Behälter auf eine vorgegebene Temperatur erwärmt und bei gleicher Temperatur konstant warm gehalten wird.

5. Verfahren nach Anspruch 1, dadurch gekennzeichnet, dass das Produkt in Einheitsmengen unterteilt in den Behälter eingeführt wird, wobei gleichzeitig eine oder mehr als eine Einheitsmenge gleichzeitig mit Sattdampf von 110° bis 165° Celsius während einer vorgegebenen Zeitdauer behandelt wird.

6. Verfahren nach Anspruch 1, dadurch gekennzeichnet, dass das Produkt auf eine vorgegebene Temperatur abgekühlt wird.

7. Vorrichtung zur Durchführung des Verfahrens nach einem der Ansprüche 1 bis 6 bestehend aus einem Behälter und mindestens eines Gefässes, dadurch gekennzeichnet dass der Behälter 1, 1.1, 1.2 ein autoklavartiger auf- und zuschliessbarer Behälter ist zur Aufnahme des mindestens einen Gefässes 38, 38.1, 38.2, welches mit einem wasseraufnahme- und wasserabgabefähigen sowie dampfdurchlässigen Gewebe zur Aufnahme einer Produktschicht versehen ist.

8. Vorrichtung nach Anspruch 7, dadurch gekennzeichnet, dass zur Abdeckung der- Produktschicht ein wasseraufnahme- und wasserabgabefähiges sowie dampfdurchlässiges Gewebe vorgesehen ist.

9. Vorrichtung nach Anspruch 7 und 8, dadurch gekennzeichnet, dass unterhalb des die Schicht aufnehmenden und oberhalb des die Schicht abdeckenden Flächengebildes ein Raum vorgesehen ist.

10. Vorrichtung nach Anspruch 7 und 8 dadurch gekennzeichnet, dass mehrere Gefässe 38.1 zu einem Transportband (67.1, resp.67.2) zusammengefasst sind und der Behälter 1.1 derart konzipiert ist, dass dieser jeweils ein Gefäss nach dem andern aufnimmt.

11. Vorrichtung nach Anspruch 7 und 8 dadurch gekennzeichnet, dass im weiteren Deckelrahmen (55, 55.1, 55.2) vorgesehen sind, innerhalb welchen das genannte Flächengebilde eingezogen ist, wobei dieser Rahmen auf einen unteren Gefässrahmen (60, 60.1, 60.2) deckungsgleich aufsetzbar ist.

12. Vorrichtung nach Anspruch 10 und 11, dadurch gekennzeichnet, dass der Gefässrahmen (60.1) und der Deckelrahmen (55) derart ausgebildet sind, dass diese Rahmen im Betrieb eine Dicht:ung bilden

13. Vorrichtung nach Anspruch 11, dadurch gekennzeichnet, dass mehrere Gefässrahmen (38.2, 55.2) gestapelt sind und Distanzstücke (94) freien Dampfzufluss zu den Gewebeflächen sowie Bilden des Unterdruckes ermöglichen.

14. Vorrichtung nach Anspruch 7, dadurch gekennzeichnet, dass die Mittel für das Steuern des Dampfes und die Mittel für das Steuern des Vakuums eine Steuerung mit folgenden Steuerelementen, welche durch folgende Steuerschritte gesteuert werden, umfassen:
a. Öffnen eines Steuerventils (32) der Vakuumerzeugungsanlage zum erzeugen und halten einer Unterdruckes im Gehäuse (1, 1.1, 1.2) während einer vorgegebenen Zeitdauer.
b. Schliessen des vorgenannten Steuerventiles (32)
c. Öffnen eines Steuerventiles (18) der Dampferzeugeranlage für den Einlass des Dampfes in das evakuierte Gefäss (1, 1.1, 1.2) während einer vorgegebenen Zeitdauer.
d. Schliessen des unter Punkt c. vorgenannten Ventiles (18) nach Ablauf der vorgenannten Zeitdauer und Öffnen eines Dampfablassventiles (34) für das Entspannen des Dampfdruckes im Gefäss (1, 1.1, 1.2) durch Auslass des Dampfes und anschliessend erneutes Schliessen.
e. Öffnen des Steuerventiles (32) der Vakuumerzeugeranlage zur Erzeugung eines erneuten Unterdruckes im Gefäss zur Kühlung des Produktes während einer vorgegebenen Zeitdauer und erneutes Schliessen dieses letztgenannten Steuerventiles (32) nach Ablauf der vorgenannten Zeitdauer.
f. Öffnen des Dampfablassventiles (34) für das Aufheben des Unterdruckes im Gefäss (1, 1.1, 1.2)

## Claims

1. Method for sterilising a pourable product by means of the process steps :
- A predefined quantity of product to be sterilised is fed into a container
- Steam of a predefined temperature and predefined pressure is injected into the container for a predefined period
- The steam pressure is released by discharging into the atmosphere
- The container is subjected to negative pressure for the purpose of cooling the product
- For the purpose of evacuating the air in the container, the latter is subjected to negative pressure both before and after introduction of the product and before injection of the steam
characterized in that the product is first introduced into the container as a layer of predefined thickness within a vessel.

2. Method according to Claim 1, characterized in that the product layer lies on a woven fabric which is capable of absorbing and releasing water and is permeable to steam.

3. Method according to Claim 2, characterized in that the product layer is additionally covered by a woven fabric which is capable of absorbing and releasing water and is permeable to steam.

4. Method according to Claim 1, characterised in that the container is heated to a predefined temperature and is kept warm, at a constant temperature.

5. Method according to Claim 1, characterised in that the product is introduced into the container divided into unitary quantities, with one or more unitary quantities being treated simultaneously with saturated steam of 110° to 165° Celsius for a predefined period.

6. Method according to Claim 1, characterized in that the product is cooled to a predefined temperature.

7. Device for execution of the method according to any one of Claims 1 to 6, consisting of a container and one or more vessels, characterized in that the container 1, 1.1, 1.2 is an autoclave-type container, which can be opened and closed, for holding the vessel, or vessels, 38, 38.1, 38.2 which possesses a woven fabric, capable of absorbing and releasing water and permeable to steam, for the purpose of holding a product layer.

8. Device according to Claim 7, characterized in that there is a woven fabric, which is capable of absorbing and releasing water and is permeable to steam, for covering the product layer.

9. Device according to either of Claims 7 or 8, characterized in that there is a space below the surface body holding the layer and above that covering the layer.

10. Device according to either of Claims 7 or 8, characterized in that several vessels 38.1 are combined to form one conveyor belt (67.1 or 67.2) and the container 1.1 is designed so that it receives one vessel after another.

11. Device according to either of Claims 7 or 8, characterized in that there are also cover frames (55, 55.1, 55.2) within which is inserted the said surface body, this frame being such that it can be placed congruently upon a lower vessel frame (60, 60.1, 60.2).

12. Device according to either of Claims 10 or 11, characterized in that the vessel frame (60.1) and the cover frame (55) are constructed so that, in operation, these frames form a seal.

13. Device according to Claim 11, characterized in that several vessel frames (38.2, 55.2) are stacked together and distance pieces (94) allow steam to flow freely to the woven fabric surfaces and permit creation of the negative pressure.

14. Device according to Claim 7, characterized in that the means for controlling the steam and the means for controlling the negative pressure include a controller with the following control elements which are controlled by the following control steps :
a. Opening of a control valve (32) of the vacuum generating system for the purpose of generating and maintaining a negative pressure within the housing (1, 1.1, 1.2) for a predefined period.
b. Closing of the said control valve (32).
c. Opening of a control valve (18) of the steam generating system for admission of the steam into the evacuated vessel (1, 1.1, 1.2) during a predefined period.
d. Closing of the valve (18) mentioned in Point c following expiry of the said period of time and opening of a steam discharge valve (34) for the purpose of releasing the steam pressure within the vessel (1, 1.1, 1.2) by discharging the steam, followed by reclosure.
e. Opening of the control valve (32) of the vacuum generating system to generate a negative pressure within the vessel for the purpose of cooling the product for a predefined period and reclosure of this latter control valve (32) following expiry of the said period of time.
f. Opening of the steam discharge valve (34) for the purpose of neutralizing the negative pressure in the vessel (1, 1.1, 1.2).

## Revendications

1. Procédé pour stériliser un produit de déversement, avec les pas de procédé suivants:
- Introduction d'une quantité prédéterminée du produit à stériliser dans un conteneur.
- Insufflation de vapeur à température et pression prédéterminées dans le conteneur pendant un laps de temps prédéterminé.
- Détente de la vapeur par échappement dans l'atmosphère.
- Mise du conteneur sous vide pour refroidir le produit.
- Le conteneur, après l'introduction du produit et avant l'introduction du produit et avant l'introduction de la vapeur pour l'évacuation de l'air se trouvant à l'intérieur, est à placer sous vide, caractérisé par le fait que le produit est déjà introduit en couche d'une épaisseur prédéterminée dans un récipient dans le conteneur.

2. Procédé selon droit 1, caractérisé par le fait que la couche de produit se trouve sur un tissu susceptible d'absorber et de restituer l'eau et perméable à la vapeur.

3. Procédé selon droit 2, caractérisé par le fait qu'en plus, la couche de produit est recouverte par un tissu perméable à la vapeur, susceptible d'absorber et de restituer l'eau.

4. Procédé selon droit 1, caractérisé par le fait que le conteneur est chauffé à une température prédéterminée et maintenu constamment à la même température.

5. Procédé selon droit 1, caractérisé par le fait que le produit est introduit dans le conteneur partagé en quantités homogènes, à l'occasion de quoi une ou plusieurs de ces quantités sont traitées simultanément avec de la vapeur saturée de 110° à 165° Celsius pendant un laps de temps prédéterminé.

6. Procédé selon droit 1, caractérisé par le fait que le produit est refroidi à une température prédéterminée.

7. Dispositif pour le déroulement du procédé selon l'un des droits 1 à 6, composé d'un conteneur et au moins d'un recipient, caractérisé par le fait que le conteneur 1, 1.1, 1.2, est un conteneur du type autoclave, susceptible d'être ouvert et fermé à volonté pour la réception d'au moins un récipient 38, 38.1, 38.2, lequel est muni d'un tissu susceptible d'être imbibé d'eau et de la restituer, perméable à la vapeur, pour absorber une couche de produit.

8. Dispositif selon droit 7, caractérisé par le fait que, pour recouvrir la couche de produit, il est prévu un tissu susceptible d'absorber l'eau et de la restituer ainsi que perméable à la vapeur.

9. Dispositif selon droits 7 et 8, caractérisé par le fait qu'un espace est prévu en-dessous de la couche de tissu imbibée et au-dessus de la couche de tissu recouvrante.

10. Dispositif selon droits 7 et 8, caractérisé par le fait que plusieurs récipients 38.1 se trouvent sur une bande de transport (resp. 67.1 et 67.2) bien serrés ensemble et que le conteneur 1.1 est conçu de façon à réceptionner un récipient après l'autre.

11. Dispositif selon droits 7 et 8, caractérisé par le fait que, en outre, des cadres de couvercles (55, 55.1, 55.2) sont prévus, à l'intérieur desquels le tissu cite est infilé, de telle sorte que ce cadre puisse coïncider avec le cadre inférieur (60, 60.1, 60.2) du récipient.

12. Dispositif selon droits 10 et 11, caractérisé par le fait que le cadre du récipient (60.1) et le cadre du couvercle (55) sont conçus de telle sorte qu'ils forment une étanchéité pendant l'opération.

13. Dispositif selon droit 11, caractérisé par le fait que plusieurs cadres de récipient (38.2, 55.2) sont empilés et que les pièces d'écartement (94) permettent la libre admission de la vapeur à la surface du tissu ainsi que la formation du vide.

14. Dispositif selon droit 7, caractérisé par le fait que les moyens de commande de la vapeur et les moyens de commande du vide comprennent un système de commande avec les éléments suivants, lesquels sont commandés d'après les pas suivants:
a. ouverture d'une vanne-pilote (32) de l'installation de production de vide pour produire et maintenir le vide dans le boîtier (1, 1.1, 1.2) pendant un laps de temps prédéterminé.
b. fermeture de la vanne-pilote mentionnée (32).
c. ouverture d'une vanne-pilote (18) de l'installation de production de vapeur pour l'admission de la vapeur dans le récipient évacué (1, 1.1, 1.2) pendant un laps de temps prédéterminé.
d. fermeture de la vanne (18) déjà mentionnée au point c. après l'écoulement du laps de temps prévu et ouverture d'une soupape d'évacuation de vapeur (34) pour la détente de la pression de la vapeur dans le conteneur (1, 1.1, 1.2) par la sortie de la vapeur et finalement, nouvelle fermeture.
e. ouverture de la vanne-pilote (32) de l'installation de production de vide pour la production d'un nouveau vide dans le récipient pour le refroidissement du produit pendant un laps de temps prédéterminé et nouvelle fermeture de la vanne déjà mentionnée (32) après écoulement du laps de temps prévu.
f. ouverture de la soupape d'évacuation de la vapeur (34) pour la compensation du vide dans le conteneur (1, 1.1, 1,2)
